Europäisches Patentamt

European Patent Office

Office européen des brevets

(19)

(11) Numéro de publication: **0 248 749**
**A2**

(12) **DEMANDE DE BREVET EUROPEEN**

(21) Numéro de dépôt: 87430022.1

(22) Date de dépôt: 25.05.87

(51) Int. Cl.⁴: **G 02 B 5/26**
G 02 B 5/28, G 02 F 1/13

(30) Priorité: 27.05.86 FR 8607685

(43) Date de publication de la demande:
09.12.87 Bulletin 87/50

(84) Etats contractants désignés:
AT BE CH DE ES FR GB GR IT LI LU NL SE

(71) Demandeur: **Aguilera, Serge**
**22, boulevard d'Athènes**
**F-13001 Marseille (FR)**

(72) Inventeur: **Aguilera, Serge**
**22, boulevard d'Athènes**
**F-13001 Marseille (FR)**

(74) Mandataire: **Marek, Pierre**
**28 & 32 rue de la Loge**
**F-13002 Marseille (FR)**

(54) Procédé et dispositif de réfraction et de polarisation de la lumière.

(57) Procédé et dispositif de réfraction et de polarisation de la lumière, caractérisés par le fait que la lumière ambiante (L) est réfractée et polarisée au moyen d'un dispositif réfracteur et polariseur principalement constitué de quatre filtres interférentiels (1, 2, 3, 4) à bandes passantes différentes, et d'une feuille ou couche de cristaux liquides (5) constitués, de préférence et avantageusement, par des cristaux cholestériques thermochromiques ou par des cristaux nématiques hélicoïdaux thermochromiques, lesdits filtres et feuilles de cristaux liquides étant accolés ou superposés les uns à la suite des autres. Ce dispositif peut être utilisé pour le traitement thérapeutique de maux et douleurs divers.

Fig.2

EP 0 248 749 A2

## Description

Procédé et dispositif de réfraction et de polarisation de la lumière.

La présente invention concerne un procédé et un dispositif de réfraction et de polarisation de la lumière.

Ce procédé et ce dispositif sont susceptibles de diverses applications industrielles et scientifiques, et on décrit ci-après un exemple d'application très avantageux mais nullement limitatif, au domaine médical.

Si on connait un certain nombre d'applications industrielles et scientifiques de la réfraction et de la polarisation de la lumière, le champ inexploré des applications possibles demeure certainement extrêmement vaste, tandis que l'application de ces phénomènes à la médecine ressort toujours du domaine expérimental.

Il a par exemple été constaté (FR-A-2 453 654) que pour une certaine orientation de son axe de polarisation et moyennant une intensité lumineuse suffisante, la lumière polarisée possède un effet de surface sur la peau, celle-ci devenant moins sensible ou plus sensible au contact d'une pointe piquante. Toutefois, cet effet de la lumière polarisée faisant appel au système nerveux cérébrospinal et aboutissant à une modification de la sensibilité très superficielle ou "épicritique", ne peut se propager en profondeur, car dès qu'elle a commencé à traverser la couche superficielle de la peau, la lumière perd sa polarisation et redevient "banale". La lumière polarisée à l'aide des procédés et dispositifs de polarisation actuels n'agit que très faiblement sur un versant du système nerveux et n'a donc pu être utilisée à des fins thérapeutiques.

Dans le domaine de la médecine, le problème de la transformation de la lumière de façon à lui conférer, à la fois, des caractéristiques lui permettant de pénétrer profondément dans l'organisme, et des propriétés curatives, demeure irrésolu.

Le procédé et le dispositif qui font l'objet de la présente invention apportent une solution à ce problème et permettent d'obtenir une lumière réfractée et polarisée dont les caractéristiques lui confèrent des effets thérapeutiques certains et durables vis à vis de nombreux maux, douleurs et affections.

Le procédé selon l'invention est remarquable par le fait que la lumière ambiante est réfractée et polarisée au moyen d'un dispositif réfracteur et polariseur principalement constitué de quatre filtres interférentiels à bandes passantes différentes, et d'une feuille ou couche de cristaux liquides lesquels sont, de préférence, constitués par des cristaux cholestériques thermochromiques ou par des cristaux nématiques hélicoïdaux thermochromiques.

Le dispositif suivant l'invention se caractérise par le fait qu'il comprend principalement quatre filtres interférentiels se différenciant par leurs bandes passantes différentes les unes des autres, et une couche ou feuille de cristaux liquides lesquels sont, de préférence, constitués par des cristaux cholestériques thermochromiques ou par des cristaux nématiques hélicoïdaux thermochromiques, lesdits filtres interférentiels et couche de cristaux étant disposés les uns à la suite des autres.

D'une manière générale, le procédé et le dispositif selon l'invention ont pour avantage de pouvoir être mis en oeuvre à l'aide d'un système de réfraction et de polarisation très simple et ne nécessitant aucune source de lumière artificielle particulière.

Dans le domaine médical, ce procédé et ce dispositif permettent de produire un faisceau lumineux réfracté et polarisé ayant des effets thérapeutiques dans le traitement d'un grand nombre de maux, douleurs et affections, principalement non traumatiques.

Dans cette application, le procédé et le dispositif suivant l'invention offrent un grand nombre d'avantages par rapport à la médication traditionnelle :
- suppression des diverses contraintes liées à l'introduction de substances médicamenteuses dans l'organisme ;
- absence de toxicité, d'accoutumance, d'allergie et d'effets secondaires ;
- application unique ou peu fréquente du traitement (une seule fois dans les crises aiguës et une fois tous les 15 jours ou de manière plus espacée pour les douleurs chroniques) ;
- courte durée de traitement (de quelques secondes à 15 minutes, dans la plupart des cas) ;
- traitement indolore ;
- facilité d'application du traitement, laquelle ne nécessite aucune connaissance particulière ;
- facilité de mise en oeuvre du procédé et du dispositif quel que soit le lieu et en toutes circonstances ;
- le dispositif est inaltérable, dans les conditions normales de température ;
- l'utilisation répétée du dispositif n'atténue en rien ses propriétés ;
- sa durée de fonctionnement est quasiment illimitée ;
- l'application du traitement ne présente aucun inconvénient et n'impose aucune contrainte fâcheuse.

Les buts, caractéristiques et avantages susmentionnés, et d'autres encore, ressortiront mieux de la description qui suit et des dessins annexés dans lesquels :

La figure 1 est une vue à caractère schématique du dispositif de réfraction et de polarisation de la lumière selon l'invention.

La figure 2 est une vue à caractère schématique illustrant l'application du procédé et du dispositif de l'invention au traitement d'une douleur céphalique.

Les figures 3 à 6 sont des représentations schématiques des quatre filtres interférentiels de ce dispositif.

On se reporte auxdits dessins pour décrire un exemple intéressant, quoique nullement limitatif, d'exécution du dispositif et de mise en oeuvre du procédé de réfraction et de polarisation de la lumière selon l'invention.

Ce procédé agit par l'interaction de la lumière et de la matière et, dans l'application de l'invention au domaine médical, par l'interaction de la lumière avec le milieu biologique, à partir d'une réflexion de la lumière ambiante obtenue au moyen d'un dispositif à la fois réfractant et polarisant permettant de sélectionner les longueurs d'onde des quantas lumineux.

On sait que la plupart des composants essentiels du milieu biologique sont constitués de cristaux cholestériques (membranes cellulaires, ADN, gaines de myéline, substance blanche du cerveau, tissu osseux, etc...), substances mésomorphes aux propriétés optiques connues, mais dont les potentialités reconnues n'ont pas encore trouvé d'applications thérapeutiques.

On sait également que dans le milieu biologique, les différentes structures constituant l'"anatomie" sont disposées de façon stratifiée et jouent en quelque sorte le rôle de filtres.

On a donc cherché à créer entre le dispositif de réfraction et de polarisation de la lumière selon l'invention et le milieu biologique, une analogie structurelle et une analogie de propriétés.

Ce dispositif comprend essentiellement quatre filtres interférentiels 1, 2, 3, 4, et une feuille ou couche de cristaux liquides 5 lesquels sont, de préférence, et avantageusement constitués par des cristaux cholestériques thermochromiques donnant les meilleurs résultats, ou des cristaux nématiques hélicoïdaux thermochromiques.

Les filtres interférentiels 1, 2, 3, 4 sont des filtres optiques connus ne laissant passer, de la lumière, qu'une ou plusieurs bandes du spectre visible ou dans l'infrarouge, et ils se différencient les uns des autres par le nombre et/ou les caractéristiques de leurs bandes passantes.

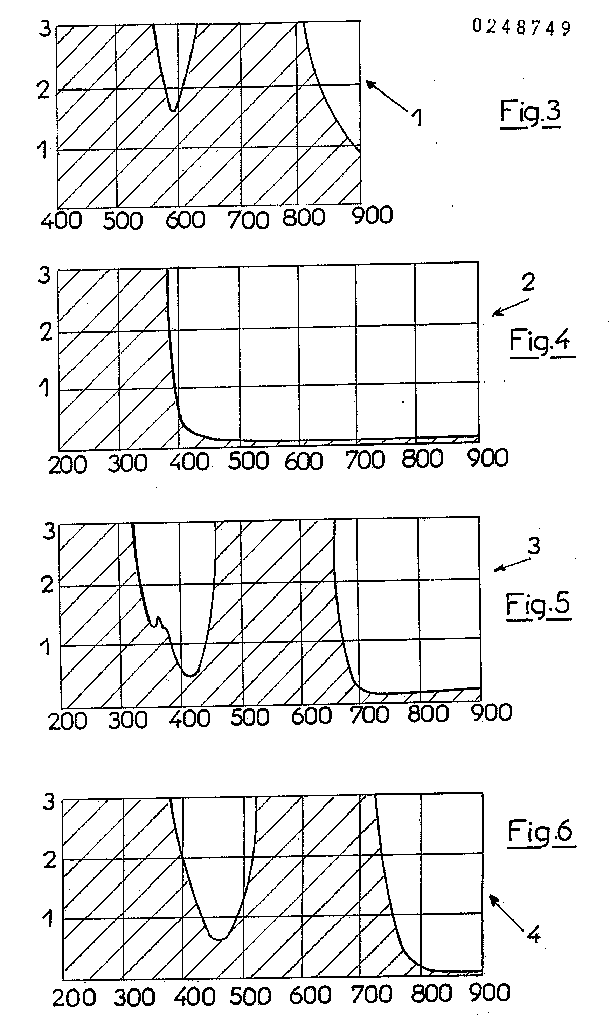

Les figures 3 à 6 représentent, schématiquement, quatre filtres interférentiels avantageusement utilisables pour la mise en oeuvre du procédé et du dispositif selon l'invention, les longueurs d'ondes en nanomètres étant portées en abscisse sur les schémas desdites figures, tandis que les chiffres portés en ordonnée indiquent la densité optique desdits filtres.

Le filtre 1 comporte deux bandes passantes, l'une comprise autour de 600 nm, la seconde entre 800 et 900 nm.

Le filtre 2 comporte une unique bande passante comprise entre 400 et 900 nm.

Le filtre 3 comporte deux bandes passantes, l'une comprise autour de 400 nm, la seconde entre 650 et 900 nm.

Le filtre 4 comporte également deux bandes passantes, l'une comprise entre 400 et 500 nm, l'autre entre 700 et 900 nm.

Il n'est pas exclu que l'on puisse remplacer les quatre filtres susmentionnés, par un filtre interférentiel unique présentant, à lui seul, les mêmes caractéristiques optiques que celles de ces quatre filtres réunis.

De manière avantageuse, la feuille ou couche de cristaux liquides 5 est constituée par des cristaux cholestériques thermochromiques à rotation droite ou par des cristaux nématiques hélicoïdaux thermochromiques à rotation droite.

Cette feuille ou couche de cristaux liquide est, par exemple, constituée par un mélange d'un certain pourcentage de cristaux (environ 30 %) avec un liant, ce mélange étant étalé et collé au moyen de ce liant, sur une feuille de matière plastique mince, transparente et neutre. La feuille de cristaux liquides ainsi réalisée peut être stockée et découpée, ce qui facilite la fabrication industrielle du dispositif selon l'invention.

Les filtres interférentiels 1, 2, 3, 4 et la feuille ou couche de cristaux liquides 5 sont superposés ou accolés suivant un ordre indifférent.

Toutefois, la feuille ou couche de cristaux liquides 5 tels que cristaux cholestériques thermochromiques ou cristaux nématiques hélicoïdaux thermochromiques, est, de préférence, disposée le plus près possible de la surface de sortie S du faisceau lumineux réfracté et polarisé F. La feuille ou couche de cristaux liquides 5 est, par exemple, disposée entre un filtre interférentiel 1 formant la surface de sortie S du faisceau lumineux réfracté et polarisé, et l'ensemble constitué par les trois autres filtres interférentiels 2, 3, 4, accolés les uns à la suite des autres.

De manière également très avantageuse, le dispositif de réfraction et de polarisation de la lumière selon l'invention comporte encore un système réfléchissant 6 placé à l'arrière de l'ensemble constitué par les filtres interférentiels 1, 2, 3, 4 et la feuille ou couche de cristaux liquides 5, c'est-à-dire à l'opposé de la surface de sortie S du faisceau lumineux réfracté et polarisé.

Ce système réfléchissant est, par exemple, constitué, de manière connue en soi, par une feuille ou plaque métallique ou autre, revêtue d'une couche de micro-billes de verre.

Le système réfléchissant 6 n'est pas indispensable. Il permet d'éliminer la transparence et de ne conserver que la réflexion de la lumière, en rotation gauche dans ce cas. La présence de ce système réfléchissant permet toutefois d'accroître les effets thérapeutiques du procédé et du dispositif.

Dans l'application au domaine médical, le procédé et le dispositif selon l'invention permettent d'appliquer un faisceau lumineux réfracté et polarisé sur n'importe quelle partie du corps humain.

Il suffit, pour celà, de placer la personne à traiter dans un endroit convenablement éclairé, par la lumière du jour ou par une source ordinaire de lumière artificielle, et de disposer le dispositif de réfraction et de polarisation de la lumière précédemment décrit, soit à proximité de la zone malade ou douloureuse du corps de ladite personne, soit à proximité d'une zone réflexe commandant ladite zone malade; le dispositif étant par exemple placé à une distance de l'ordre de 5 à 10 cm par rapport aux zones susmentionnées. La surface de sortie S du dispositif est orientée face à la zone à traiter de manière que la lumière ambiante L soit réfractée et polarisée par ledit dispositif et que l'énergie lumineuse F engendrée par ce dernier soit dirigée et appliquée sur ladite zone.

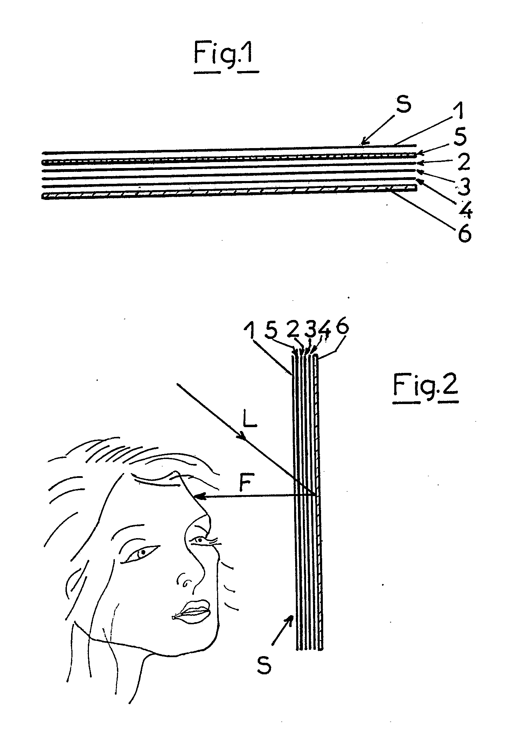

Par exemple, pour un mal de tête bénin, le dispositif de réfraction et de polarisation doit être disposé, soit à proximité du crâne de la personne malade (figure 2), soit à proximité de l'articulation métacarpophalangienne du 5ème doigt (qui correspond au 3ème point du méridien de l'intestin grèle en acupuncture). Dans ce cas, la sédation de la douleur est quasi instantannée.

La durée d'application du rayonnement ou faisceau lumineux réfracté est généralement de l'ordre de quelques secondes à 5 minutes, suivant les cas, le temps moyen d'application étant de 5 minutes environ.

Lorsqu'aucun effet thérapeutique ne se manifeste après un temps d'application de l'ordre de 5 à 10 minutes, on peut arrêter cette application, car le traitement n'est vraisemblablement pas indiqué pour le mal concerné.

Lorsque l'effet thérapeutique se fait sentir (généralement assez rapidement, après un temps d'application de l'ordre de quelques secondes à 30 ou 40 secondes), on doit continuer l'application aussi longtemps que l'effet thérapeutique s'accroit. Quant l'effet optimal est obtenu, l'application doit être poursuivie durant quelques secondes.

Au vu des expériences et des tests effectués, il semble que le procédé et le système optique thérapeutique selon l'invention agissent :
- par une sorte d'effet photoélectrique ;
- par un champ photo-électronique ;
réagissant avec les champs biologiques de même nature de l'organisme.

Ce procédé et ce système de réfraction et de polarisation de la lumière produisent un effet curatif définitif ou durable dans le traitement de nombreux maux, douleurs ou affections, principalement d'origine non traumatique, la durée de l'effet thérapeutique étant variable suivant les indications.

Ce procédé et ce dispositif peuvent, par exemple, être utilisés avec succès dans le traitement des maux ou troubles suivants :  .

- 1) lumbagos : l'effet thérapeutique se manifeste très rapidement après une application directe d'une durée de quelques secondes à quelques minutes au-dessus de la colonne vertébrale ; la durée de l'effet thérapeutique va de 24 heures à la guérison définitive.

- 2) Algies veineuses des membres inférieurs : l'application se fait sous la plante des pieds, pendant une durée comprises entre 2 minutes et 5 minutes ; deux à trois applications identiques à quelques jours d'intervalle permettant d'obtenir une sédation des douleurs pendant plusieurs mois (3 mois et plus).

- 3) Céphalées : l'application se fait autour du crâne; la durée d'action va de quelques heures à la disparition définitive de la douleur, suivant le type de céphalée. Dans le cas de céphalée banale, l'effet thérapeutique est immédiat et définitif.

- 4) aphtoses buccales : l'effet thérapeutique est immédiat et définitif après une application d'une durée de quelques secondes à 5 minutes.

- 5) troubles artériels cérébraux par ralentissement du flux sanguin : l'effet thérapeutique est probant après 5 à 10 minutes d'application péricranienne, cet effet se manifestant immédiatement par une sensation d'éclaircissement et de meilleure vue. La durée de l'effet curatif varie de quelques heures à plusieurs semaines, suivant les cas ; la répétition du traitement augmentant la durée des effets thérapeutiques dans le temps.

- 6) Algies ayant une origine sympatique (par exemple : douleurs séquellaires post-fracture de cheville) : l'application du traitement assure la disparition de la douleur d'une façon quasi instantanée et définitive.

- 7) Torticolis : l'action du traitement est instantanée et définitive pour un torticolis aigu, après 15 minutes d'application.

- 8) Brûlures superficielles : la disparition de la douleur est définitive après une application d'une durée d'une trentaine de secondes à 2 ou 3 minutes.

- 9) Certains problèmes digestifs (spasmes, etc.) : l'application est faite sous la plante des pieds et son effet thérapeutique est variable, ainsi que la durée d'action du traitement laquelle varie de quelques heures à plusieurs jours.

- 10) Rhumes de cerveau et rhumes allergiques (rhumes des foins) : l'application du traitement produit une action scurative rapide et de longue durée.

**Revendications**

1. - Procédé de réfraction et de polarisation de la lumière, caractérisé par le fait que la lumière ambiante (L) est réfractée et polarisée au moyen d'un dispositif réfracteur et polariseur principalement constitué de quatre filtres interférentiels (1, 2, 3, 4) à bandes passantes différentes, et d'une feuille ou couche de cristaux liquides (5) lesquels sont, de préférence et de manière avantageuse constitués par des cristaux cholestériques thermochromiques ou par des cristaux nématiques hélicoïdaux thermochromiques, lesdits filtres et feuilles étant accolés ou superposés les uns à la suite des autres.

2. - Procédé selon la revendication 1, caractérisé en ce qu'un système réfléchissant (6), par exemple constitué d'une feuille revêtue d'une couche de micro-billes de verre, est placé en arrière du dispositif réfracteur et polariseur (1-2-3-4-5).

3. - Procédé suivant l'une des revendications 1 ou 2, caractérisé en ce que la feuille ou couche de cristaux liquides (5) tels que cristaux cholestériques thermochromiques ou cristaux nématiques hélicoïdaux thermochromiques est disposée le plus près possible de la surface de sortie (S) du faisceau lumineux réfracté et polarisé (F) et, par exemple, entre un filtre interférentiel (1) formant ladite surface de sortie (S) et l'ensemble constitué par les trois autres filtres interférentiels (2, 3, 4).

4. - Procédé selon l'une quelconque des revendications 1 à 3, caractérisé en ce qu'on utilise, pour constituer le dispositif réfracteur et polariseur (1-2-3-4-5), une feuille ou couche de cristaux cholestériques thermochromiques (5) à rotation droite ou une feuille ou couche de cristaux nématiques hélicoïdaux thermochromiques (5) à rotation droite.

5. - Procédé suivant l'une quelconque des revendications 1 à 4, caractérisé en ce que l'on utilise, pour constituer le dispositif réfracteur et polariseur (1-2-3-4-5) :
- un premier filtre interférentiel (1) comportant deux bandes passantes dont l'une est comprise autour de 600 nm et l'autre entre 800 et 900 nm ;
- un deuxième filtre interférentiel (2) comportant une unique bande passante comprise entre 400 et 900 nm ;
- un troisième filtre interférentiel (3) comportant deux bandes passantes dont l'une est comprise autour de 400 nm et l'autre entre 650 et 900 nm ;
- un quatrième filtre interférentiel (4) comportant deux bandes passantes dont l'une est comprise entre 400 et 500 nm et l'autre entre 700 et 900 nm.

6. - Dispositif de réfraction et de polarisation de la lumière, caractérisé en ce qu'il comprend principalement quatre filtres interférentiels (1, 2, 3, 4) se différenciant par leurs bandes passantes différentes les unes des autres, et une feuille ou couche de cristaux liquides (5) constitués, de préférence et avantageusement, par des cristaux cholestériques thermochromiques ou par des cristaux nématiques hélicoïdaux thermochromiques, lesdits filtres interférentiels et couche de cristaux liquides étant disposés les uns à la suite des autres.

7. - Dispositif de réfraction et de polarisation de la lumière selon la revendication 6, caractérisé en ce qu'il comprend encore un système réfléchissant (6) disposé à l'arrière du dispositif réfracteur et polariseur (1-2-3-4-5), ce système réfléchissant étant, par exemple, constitué, de façon connue en soi, par une feuille ou plaque revêtue d'une couche de micro-billes de verre.

8. - Dispositif suivant l'une des revendications 6 ou 7, caractérisé en ce que la feuille ou couche de cristaux liquides (5) tels que cristaux cholestériques thermochromiques ou cristaux nématiques hélicoïdaux thermochromiques est disposée entre l'un des filtres interférentiels (1) constituant la surface de sortie (S) du faisceau lumineux réfracté et polarisé (F), et l'ensemble constitué par les trois autres filtres interférentiels (2, 3, 4).

9. - Dispositif selon l'une quelconque des revendications 6 à 8, caractérisé en ce que la feuille ou couche de cristaux liquides (5), est une feuille ou couche de cristaux cholestériques thermochromiques à rotation droite ou une feuille ou couche de cristaux nématiques hélicoïdaux thermochromiques à rotation droite.

10. - Dispositif suivant l'une quelconque des revendications 6 à 9, caractérisé en ce que les filtres interférentiels sont constitués par :
- un premier filtre interférentiel (1) comportant deux bandes passantes dont l'une est comprise autour de 600 nm et l'autre entre 800 et 900 nm ;
- un deuxième filtre interférentiel (2) comportant une unique bande passante comprise entre 400 et 900 nm ;
- un troisième filtre interférentiel (3) comportant deux bandes passantes dont l'une est comprise autour de 400 nm et l'autre entre 650 et 900 nm ;
- un quatrième filtre interférentiel (4) comportant deux bandes passantes dont l'une est comprise entre 400 et 500 nm et l'autre entre 700 et 900 nm.

## Fig.1

## Fig.2

0248749

Fig.3

Fig.4

Fig.5

Fig.6